# EUROPEAN PATENT APPLICATION

(11) **EP 3 103 864 A1**
(43) Date of publication of application: **14.12.2016**
(21) Application number: 16174217.6
(22) Date of filing: 13.06.2016
(51) Int. Cl.: C12M 3/00, C12M 1/22, C12M 1/00, C12M 1/34

(54) **INCUBATOR**

(30) Priority: 12.06.2015 GB 201510359
(71) Applicant: Planer Plc, Sunbury, Middlesex TW16 7HD (GB)
(72) Inventor: Wilkins, Stephen, London, N8 8RA (GB); Butler, Stephen, Wokingham, Berkshire RG41 3GH (GB)
(74) Representative: Hocking, Adrian Niall

(57) **Abstract**

An incubator (10) for regulating the temperature of cell culture samples, the incubator (10) comprising: a support element (12) having a base portion (18) which has an upper lid-facing surface (28) for supporting cell culture samples; and a lid (14) which covers or substantially covers the support element (12) in an engaged condition. The base portion (18) of the support element (12) is at least in part thermally conductive at or adjacent to the lid-facing surface (28) and includes a layered heater (42) comprising: a heating element (44); and a thermal equalisation layer (48) interposed between and being in thermal communication with the heating element (44) and the base portion (18). An incubator (10) compatible with microscope interrogation of cell culture samples contained therein, a method of maintaining thermal equilibrium within cell culture samples and a method of optically interrogating cell culture samples within an incubator are also provided.

## Description

The present invention relates to an incubator, in particular but not necessarily exclusively, for incubating embryonic samples as part of in vitro fertilisation (IVF) processes. The invention further relates to a method of maintaining thermal equilibrium within cell culture samples, and to a method of optically interrogating cell culture samples within an incubator.

Incubators are well-known for keeping cell culture samples at a consistent temperature in order to promote growth. Such incubators are of particular relevance as part of the IVF process, in which it is necessary to incubate the fertilised embryos at human core body temperature, around 37°C, and within a controlled atmosphere.

The progress of the fertilised embryos should be monitored during the IVF process, and this is typically performed by removing Petri dishes in which the embryos are located from an incubator and then viewing them under a microscope. However, the problem with monitoring the embryos is the difficulty of introducing optical access without compromising the thermal retention of the incubator; opening the incubator to remove the embryos therefrom can result in rapid cooling which could kill the embryos.

Some incubators have been created which contain an integrated microscope which is capable of relaying images of the embryos to a user. However, if the microscope is integrated into the controlled environment, then the difficulty arises in effective location of the embryos within its a Petri dish, as the user has no recourse to relocate the Petri dish with respect to the microscope.

To solve this issue, incubators having integrated microscopes generally require bespoke glassware which includes a small, precisely located dimple in the base, ensuring that the embryo is in the correct position to be imaged. However, even with such an addition, the incubators still generally require the use of low magnification microscope lenses, typically x20, in order to yield the necessarily large field of view in order to locate the embryo.

Due to the need to provide a microscope which is integrally formed with the incubator, the resultant incubator is therefore not cost-effective, and the embryos cannot be interrogated using standard laboratory microscopes without removing the embryos from the incubator.

It is an object of the present invention to provide a cost-effective incubator within which cell culture samples can be maintained at a consistent and even temperature whilst also being simultaneously viewable using a standard desktop laboratory microscope.

According to a first aspect of the invention there is provided an incubator for regulating the temperature of cell culture samples, the incubator comprising: a support element having a base portion which has an upper lid-facing surface for supporting cell culture samples; and a lid which covers or substantially covers the support element in an engaged condition; the base portion of the support element being at least in part thermally conductive at or adjacent to the lid-facing surface, and including a layered heater comprising: a heating element; and a thermal equalisation layer interposed between and being in thermal communication with the heating element and the lid-facing surface of the base portion.

By providing a means of dissipating thermal energy from a heater throughout the incubator, by the use of a thermal equalisation layer, it is beneficially possible to avoid the presence of significant temperature differences between adjacent Petri dishes which would otherwise result in the cell culture samples being unevenly incubated. This advantageously minimises the risk of cell culture sample death due to over- or under-heating.

Preferably, the thermal equalisation layer may have anisotropic heat transfer properties within a plane of the lid-facing surface and perpendicular to the said plane, and in a preferred embodiment, the thermal equalisation layer may comprise at least in part graphite.

The heating element may comprise a printed circuit board having heating tracks formed thereon, and there may further comprise an electrically insulative layer interposed between the heating element and thermal equalisation layer. Optionally, the said at least in part thermally conductive base portion may be formed at least in part from aluminium.

By utilising a medium such as a graphite-based compound, in which thermal conductivity is substantially greater through the carbon honeycomb layers than it is between layers, highly anisotropic heat transfer properties can be achieved. This advantageously allows the graphite layer to thermally equalise much faster than it heats the at least in part thermally conductive base portion above, mitigating the effect of having discrete, separate heating elements which produce an uneven thermal distribution.

The support element may include a humidifier receptacle.

By providing a humidifier within the incubator, the humidity within the incubator can be maintained at a consistent and optimised level to promote embryonic growth. This allows the incubator to dispense with the standard and complex humidification bottle.

Preferably, the support element may further comprise at least one cell culture sample positioning element, and the or each cell culture sample positioning element may define a cell culture sample receptacle in which a cell culture sample is receivable. The said cell culture sample receptacles may be formed as cylindrical wells for receiving cell culture samples in Petri dishes.

An accurate location means for cell culture samples in the incubator ensures that, should the cell culture samples be optically interrogated, the position of the cell culture is predetermined, making, for instance, microscopy more straightforward.

There may be provided at least one void separating each said cell culture sample positioning element to permit user access to a cell culture sample received therein. The or each void may be dimensioned for receiving one or more user digits to engage the cell culture sample in the respective cell culture sample positioning element.

By providing a void or voids around each cell culture sample positioning element, access to the inserted cell culture samples is facilitated. For example, a pair of voids may be dimensioned so as to readily allow a user to insert their fingers around a cell culture sample in a Petri dish, simplifying insertion and extraction of the Petri dish from the receptacle.

The incubator may further comprise a lower viewing aperture through the base portion of the support element, each upper viewing aperture being associated with the or each cell culture sample positioning element, and furthermore the base portion of the support element may include a frusto-conical or substantially frusto-conical counter-bore associated with the or each viewing aperture for receiving an objective lens of a microscope.

By providing a means of viewing the cell culture samples when inserted into the incubator, the user beneficially does not need to extract the cell culture samples in order to observe them. This ensures that the precise temperature for optimum growth of the cell culture sample can be maintained. Since the thermal equalisation layer is installed in the incubator, the thermal voids created by the presence of the viewing apertures advantageously do not affect the overall thermal profile within the incubator.

Additionally, there may further comprise an upper viewing aperture through the lid associated with the or each cell culture sample positioning element.

A further aperture, co-axial with the lower viewing aperture through the cell culture sample receptacle, readily permits light to enter the incubator, beneficially ensuring that the cell culture samples can be optically interrogated without relying on artificial lighting propagated into the incubator.

Preferably, the incubator may further comprise a thermally conductive layer at or adjacent to a base-facing surface of the lid, a further thermal equalisation layer adjacent to the thermally conductive layer, and at least one thermal bridge to thermally connect the at least in part thermally conductive base portion of the support element and the thermally conductive layer, and the or each thermal bridge may be formed as part of the or each cell culture sample positioning element. The said further thermal equalisation layer may have anisotropic heat transfer properties within a plane of the lid-facing surface and perpendicular to the said plane, and may comprise at least in part graphite.

By providing a thermally conductive layer and corresponding thermal equalisation layer associated with the lid of the incubator, which is in thermal communication with the at least in part thermally conductive base portion of the support element, the need for separate heating elements integrated into the lid is removed. This advantageously reduces the bulk and manufacturing cost of the incubator, without disrupting the thermal equilibrium inside the incubator.

According to a second aspect of the invention there is provided an incubator compatible with microscope interrogation of cell culture samples contained therein, the incubator comprising: a support element for supporting cell culture samples; a lid which covers or substantially covers the support element in an engaged condition; at least one cell culture sample receptacle associated with the support element; at least one upper and lower viewing aperture respectively positioned in each of the lid and support element, the upper and lower viewing apertures defining an optical axis through the cell culture sample receptacle; and a heater associated with the support element, the heater including a thermal equalisation element which surrounds or substantially surrounds the or each lower viewing aperture to provide a uniform or substantially uniform temperature profile through the or each cell culture sample receptacle.

The thermal equalisation layer may have anisotropic heat transfer properties parallel and perpendicular to the optical axis, and may comprise at least in part graphite. The support element may include a frusto-conical or substantially frusto-conical counter-bore associated with the lower viewing aperture to receive an objective lens of a microscope.

The provision of an optical axis through the incubator allows the user to optically interrogate the cell culture sample without the need for awkward or cumbersome integrated microscopes inside the incubator. This enables the user to use a standard desktop microscope and standard high magnification objective lenses. In turn, this permits more accurate investigation into the status of the cell culture samples at any given time without needing to extract the cell culture samples from the incubator.

According to a third aspect of the invention, there is provided a method of maintaining thermal equilibrium within cell culture samples, the method comprising the steps of: a] providing an incubator, preferably in accordance with the first aspect of the invention; b] activating the heating element to indirectly heat the thermally conductive layer via the thermal equalisation layer, the thermal equalisation layer dissipating the thermal energy across its total extent; and c] heating cell culture samples within the incubator via the thermally conductive layer.

Maintenance of the thermal equilibrium within an incubator using a thermal equalisation layer ensures that isolated high temperature regions proximate to the heating element do not form within the incubator, beneficially shielding the cell culture samples from the deleterious effects of over-heating.

According to a fourth aspect of the invention, there is provided a method of optically interrogating cell culture samples within an incubator, the method comprising the steps of: a] providing an incubator having a heating element which includes a thermal equalisation element having at least one viewing aperture therethrough, the thermal equalisation element providing an even temperature through the incubator; and b] providing an optical axis through the incubator associated with the or each viewing aperture; and c] optically interrogating a cell culture sample on the optical axis inside the incubator using a microscope.

By providing an optical axis through the incubator, interrogation of cell culture samples using a standard desktop microscope is advantageously enabled, allowing the user to more accurately follow the progress of the cell culture as it grows.

The invention will now be more particularly described, by way of example only, with reference to the accompanying drawings, in which:
Figure 1 shows a perspective representation of one embodiment of an incubator in accordance with the first aspect of the invention;
Figure 2 shows a perspective representation of the incubator of Figure 1, with the lid having been removed;
Figure 3a shows a perspective cross-sectional representation through a central longitudinal axis of the support element of the incubator of Figure 1, inclusive of Petri dishes;
Figure 3b shows a magnified representation of the dashed region indicated in Figure 3a, showing a cell culture sample receptacle;
Figure 3c shows a magnified side view of the dashed region indicated in Figure 3a;
Figure 3d shows a magnified representation of the dashed region indicated in Figure 3c;
Figure 4a shows a perspective cross-sectional representation through a central longitudinal axis of the lid of the incubator of Figure 1;
Figure 4b shows a magnified representation of the dashed region indicated in Figure 4a; and
Figure 5 shows an in use thermal distribution through the incubator of Figure 1.

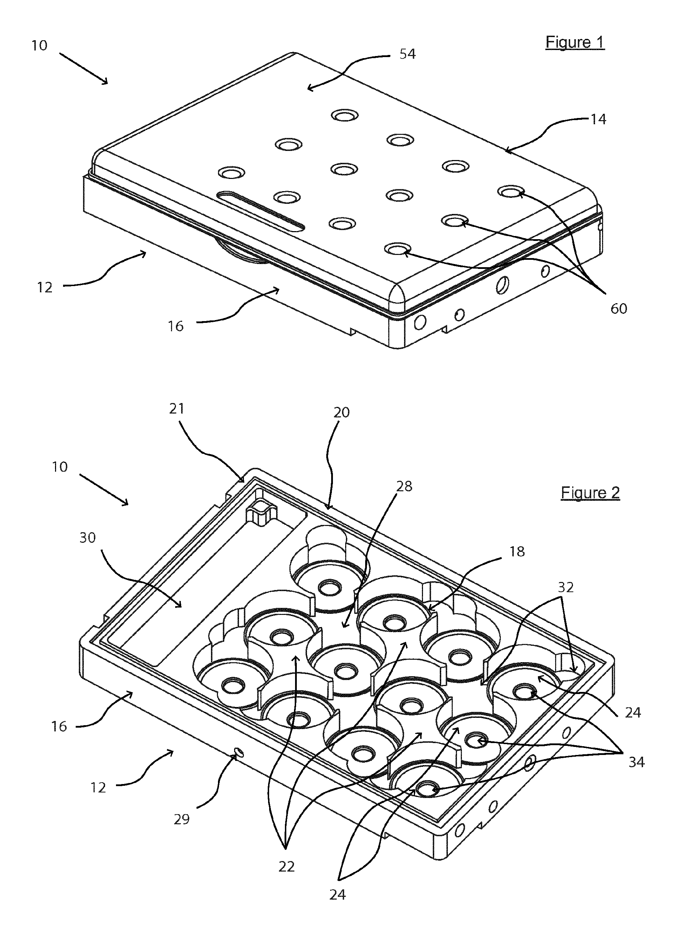

Referring to the drawings, there is shown an incubator, indicated globally at 10 for regulating the temperature of cell culture samples in an even manner such that the cell culture samples can be optically interrogated during the incubation process. The incubator 10 comprises a lower support element 12 and a lid 14 which covers or substantially covers the support element 12 in an engaged condition, as shown in Figure 1.

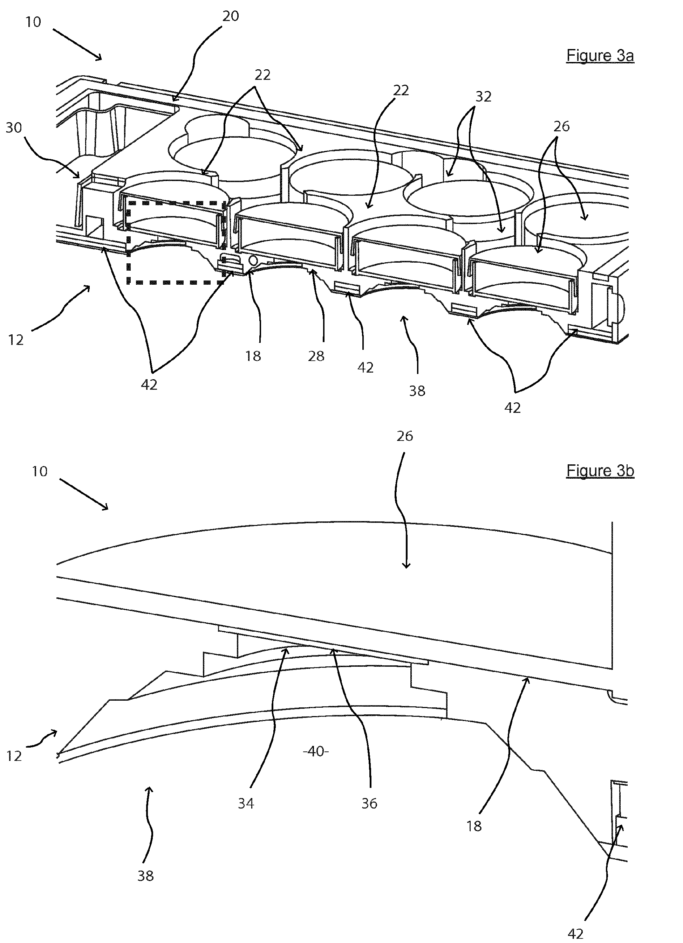

The support element 12 is preferably formed as a shallow tray 16 having a base portion 18 which extends to a tray rim 20 having a sealing groove 21 therein, with a plurality of upstanding cell culture sample positioning elements 22 extending upwardly from the base portion 18 to define a plurality of cell culture sample receptacles 24. In the illustrated embodiment, these cell culture sample receptacles 24 are formed as cylindrical wells so as to receive Petri dish samples 26 therein, and this can be seen best in Figures 2 and 3a. The bases of these wells are formed by an upper, lid-facing surface 28 of the base portion 18. Other features may also be formed into the support element 12, such as the access ports 29 illustrated for example, which allow access for a thermal probe, such as a platinum resistance thermometer, to be introduced.

The tray 16 of the support element 12 as illustrated includes a humidifier receptacle 30 which can be used to receive a water reservoir for humidifying the incubator to an optimum level for embryo growth. This humidifier receptacle 30 can be used directly as the water reservoir, if necessary, or more commonly would be supplied with a removable water reservoir.

In the depicted embodiment, the tray 16 is formed from aluminium, which is a good thermal conductor enabling an even temperature profile throughout the support element 12. However, the tray 16 could feasibly be constructed from or coated with any appropriately thermally conductive material in preference, such that the base portion 18 is at least in part thermally conductive.

The cell culture sample positioning elements 22 extend substantially from the level of the base portion 18 to the height of the rim 20 of the tray 16, each cell culture sample positioning element 22 having a substantially arcuate outer profile to receive the cell culture samples 26. Adjacent to each of the cell culture sample positioning elements 22 are two opposed voids 32 which permit user access either side of the cell culture sample receptacle 24. Such voids 32 may not be strictly necessary, though they improve the ease with which cell culture samples 26 can be inserted and removed from the incubator 10. A different number or form of void 32 will be apparent to the skilled person.

These voids 32 are dimensioned so as to permit manual extraction of cell culture samples; they allow a person to insert their digits into the cell culture sample receptacles 24 in such a manner so as to be able to engage a Petri dish 26 without accidental removal of a lid of the Petri dish 26. A single void 32 might therefore be dimensioned so as to be around 10 to 40 millimetres wide, though these dimensions can be optimised for a typical user's finger size as necessary.

Through the base portion 18 of each cell culture sample receptacle 24 is provided a lower viewing aperture 34 formed here as a circular bore having a light-transmissible plate or shim 36 positioned therein. The light-transmissible shim 36 seals the viewing aperture 34, and could be formed from, for example, glass or a transparent plastics material such as poly(methyl methacrylate).

On an underside 38 of the base portion 18 associated with each viewing aperture 34 is provided a counter-bore 40, counter-sink or complex series of counter-bores and/or counter-sinks having a generally frusto-conical shape so as to receive an objective lens of a microscope. This is best illustrated in Figure 3b. The exact shape of the counter-bore 40 may be customised depending on the shape of a corresponding objective lens of a microscope.

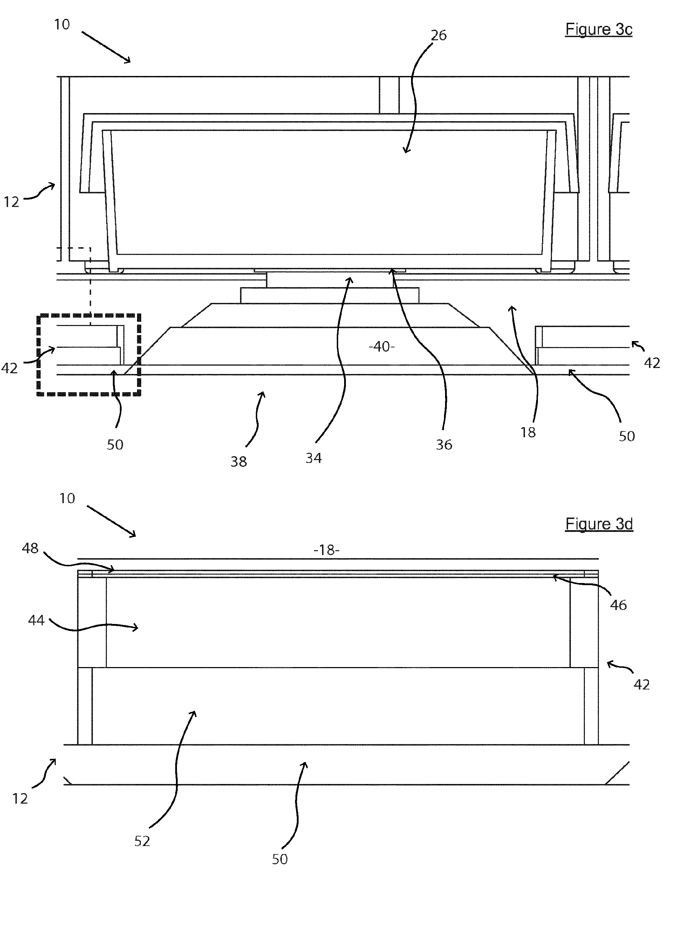

Also on the underside 38 of the base portion 18 is included a heater 42, or heaters 42 if provided separately and spaced across the base portion 18, which is formed as a layered heater 42 to provide an even or more even thermal distribution across the incubator 10. The heater 42 can be readily seen in context in Figures 3c and 3d.

The layered heater 42 comprises, in layered order, a heating element 44, an electrically insulative layer 46, a thermal equalisation layer 48, and a thermally conductive layer, which in this instance is formed by the aluminium of the base portion 18. The heater 42 is retained in place in the support element 12 by a baseplate 50, provided as a thin steel plate here which encircles the counter-bores 40 on the underside 38 of the base portion 18. The heater 42 may also be supported by a thermally insulative support 52 to provide a thermal barrier between the heating element 44 and the baseplate 50. The thermally insulative support 52 could be formed, for example, from a silicone material to achieve this aim.

The heating element 44 is in this embodiment a printed circuit board having a plurality of heated tracks thereon which produce thermal energy when a current is applied to the printed circuit board. Other heating elements are known in the art, however, such as traditional filament heaters, and such heating elements could readily be additionally or alternatively incorporated into the heater 42.

The electrically insulative layer 46 utilised here is a polyimide film which is a good thermal conductor which remains stable over a wide range of temperatures whilst remaining an electrical insulator. The electrically insulative layer 46 provides an electrical barrier between the heating element 46 and the thermal equalisation layer 48, though the electrically insulative layer 46 may be dispensed with if, for instance, the thermal equalisation layer was formed from an electrically insulating material.

The thermal equalisation layer 48 is included to disperse thermal energy from the heating element 44 in an even manner across the thermally conductive layer, that is, the base portion 18 in this embodiment, and therefore provide an even thermal profile throughout the incubator 10.

One possibility of providing the said thermal equalisation is by forming the thermal equalisation layer 48 from a material which has anisotropic heat transfer properties. The present invention utilises a graphite-based film or layer which has a far higher thermal conductivity through the plane of the film relative to the normal axis perpendicular to the plane, of the order of 100x greater thermal conductivity. One example of such a material is eGRAF RTM, available from GrafTech International of 12900 Snow Road, Parma, Ohio 44310-1012, USA. Other materials are potentially available for use as a thermal equalisation layer 48, graphene-based materials in particular, and it will be appreciated that the present invention is not intended to be limited solely to the use of eGRAF RTM.

The anisotropic heat transfer properties of the thermal equalisation layer 48 means that the material therein is able to receive thermal energy from the heating element or heating elements 44 which may be spaced-apart in an irregular manner across the support element 12. The thermal equalisation layer 48 will rapidly reach thermal equilibrium though a plane of the lid-facing surface 28 of the base portion 18 before significant heat transfer can occur to the base portion 18.

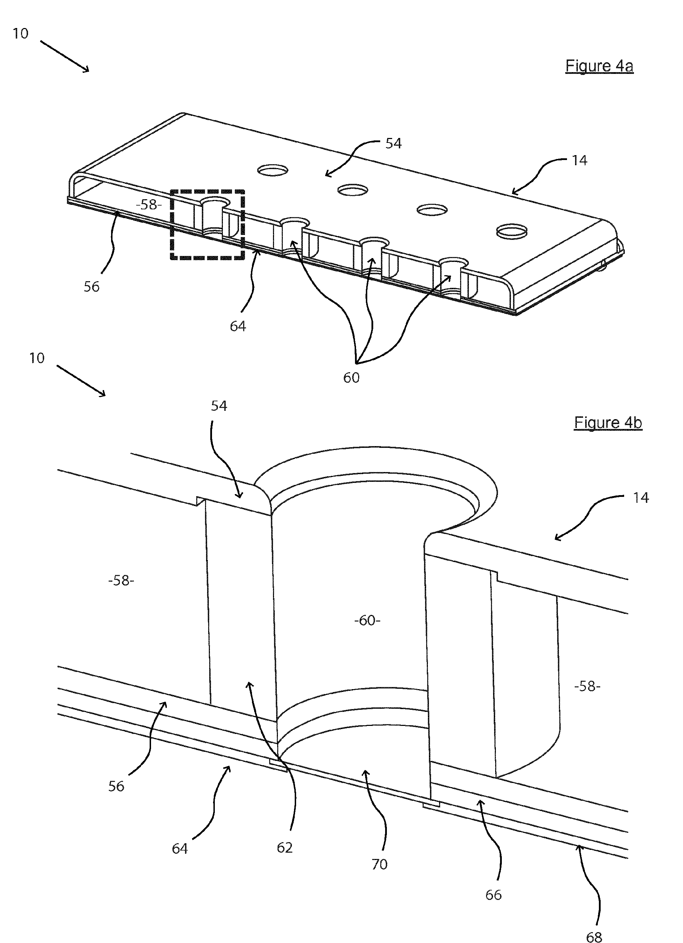

The lid 14 of the incubator 10 is preferably formed as a bilayer so as to provide thermal insulation, as shown in Figures 4a and 4b. An upper lid plate 54, formed typically from a thermoplastic material such as acrylonitrile butadiene styrene, is provided spaced apart from a lower lid plate 56, here formed as a thin steel plate, with an air-filled cavity 58 positioned therebetween.

A plurality of upper viewing apertures 60 are provided which extend through both upper and lower lid plates 54, 56, with a cylindrical spacer 62 being provided so as to define each viewing aperture 60 through the cavity 58. This spacer 62 would typically be formed from a thermally insulative plastics material such as a polyoxymethalate. The materials listed here in the construction of the lid 14 are merely described by way of example only.

On a lower, base-facing surface 64 of the lid 14 adjacent to the lower lid plate 56 is positioned a further thermal equalisation layer 66, which may be formed from the same material as the thermal equalisation layer 48 of the base portion 18, such as eGRAF RTM, but could be a different material having similar properties.

The thermal equalisation layer 66 is then retained in place by providing a thermally conductive layer 68 on the opposite side of the further thermal equalisation layer 66 to the lower lid plate 56, thereby providing a thermally conductive base-facing surface 64 of the lid 14. Again, in the depicted embodiment, the thermally conductive layer 68 is formed from aluminium for optimum heat transfer, but other thermally conductive materials could be considered. A further light-transmissible plate or shim 70 is here positioned interposed between the further thermal equalisation layer 66 and the thermally conductive layer 68 to seal the inside of the incubator 10 at each upper viewing aperture 60.

The further thermal equalisation layer 66 acts in the same manner as the said thermal equalisation layer 48, dissipating the thermal energy across the thermally conductive layer 68 to ensure an even temperature distribution within the incubator 10.

To use the incubator 10, a plurality of cell culture samples, such as embryos used in an IVF process, are inserted into Petri dishes 26 and inserted into the cell culture sample receptacles 24, being held in their horizontal positions by the cell culture sample positioning elements 22. The voids 32 assist with insertion and removal of the Petri dishes 26.

A humidifier, such as a water reservoir may be inserted into the he humidifier receptacle 30 in order to provide an optimum humidity within the incubator 10 in use. The incubator 10 can then be sealed with the lid 14 in order to complete the thermally insulated chamber for the cell culture samples, an O-ring positioned in the sealing groove 21 may form the necessary seal.

When the lid 14 is in an engaged condition with the support element 12, the cell culture sample positioning elements 22 contact the further thermally conductive layer 68 of the lid 14, thereby forming a thermal bridge between the base portion 18 and the thermally conductive layer 68. This permits ready heat transfer from the heater 42 and the base-facing surface 64 of the lid 14, via the at least in part thermally conductive base portion 18, negating the need for a separate heater to be included in the lid 14. This creates a more compact and cost-effective incubator 10 without reducing the efficiency of the incubator 10. It will be appreciated that although the cell culture sample positioning elements 22 are designed for this purpose in the depicted embodiment, dedicated thermal bridges could be provided which do not assist with the positioning of the Petri dishes 26 in use. Alternatively, although not necessarily preferable, a separate heating device may be incorporated into the lid, and this may be similar to the layered heater described above.

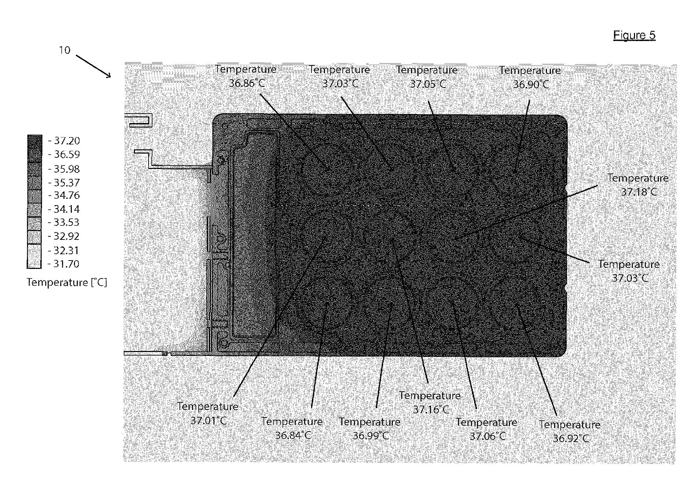

The thermal profile of incubator 10 in use is shown in Figure 5. Critically, the thermal variation within the cell culture sample receptacles 24 varies by no more than ±0.2°C either side of, for example, 37°C, ensuring that none of the cell culture samples is adversely affected by drastic temperature fluctuations despite the inclusion of an optical axis which runs through each of the cell culture sample receptacles 24, defined along the axis between upper and lower viewing apertures 34, 60.

As there is an optical axis through the cell culture sample receptacles 24, and as the underside 38 of the base portion 18 has the substantially frusto-conical counter-bores 40 formed therein, a user is able to bring an objective lens of a standard desktop microscope very close to the lower viewing aperture. In doing so, the objective lens can be positioned sufficiently close to the cell culture sample inside the incubator 10 so as to permit the use of higher magnification objective lenses, such as x40 lenses. This permits more accurate optical interrogation of the cell culture sample therein.

With the inclusion of the thermal equalisation layer 48, the upper and lower viewing apertures 34, 60 can afford to be relatively large with respect to the Petri dishes without disrupting the thermal equilibrium inside the incubator 10. This advantageously makes location of the embryo, for instance, within the Petri dish more straightforward using a microscope. Typically, an inverse microscope will be used for this purpose; since the embryos will rest at the bottom of the Petri dish 26 in which they are contained; this simplifies location and focussing of the embryo.

It will be apparent to the skilled reader that the incubator as described could be provided having a heater integrated into the lid, in addition to the support element. Alternatively, the heater could be provided solely in the lid with the thermal bridge acting to thermally conduct to the base or support element, in effect therefore being the inverse of the embodiment described above. Furthermore, the use of a thermal equalisation layer inside the incubator could be utilised without providing the viewing apertures to optically interrogate the cell culture samples, as a means of maintaining a consistent temperature inside an enclosed incubator.

Similarly, whilst the heater as described above solves the problem of how to optically interrogate cell culture samples inside an incubator without disrupting the temperature inside, it will be appreciated that a non-layered heater could be provided, providing that a sufficiently effective thermal equalisation element were included in association with whichever heating element were supplied.

Whilst the invention has thus far been described with the microscope being in close contact with the base of the incubator to view the embryos, it is plausible that counter-bores could be formed in the lid of the incubator instead, and the cell culture samples optically interrogated from above instead.

The incubator is illustrated as having cylindrical wells for receiving Petri dishes, being primarily intended for use in an IVF process, it will be appreciated that such an incubator could feasibly be used in connection with all sorts of cell culture investigations, which may utilise alternative glassware as standard. Alternative cell culture sample receptacles could clearly be provided in such instances.

It is therefore possible to provide an incubator for incubating cell culture samples, in particular embryonic samples used in IVF processes, which allows for the inspection of the cell culture samples without requiring the samples to be extracted. This can be achieved by providing an optical axis through the incubator, and using a thermal equalisation layer associated with a heater of the incubator which ensures that the thermal equilibrium is maintained for all cell culture samples within the incubator.

The words 'comprises/comprising' and the words 'having/including' when used herein with reference to the present invention are used to specify the presence of stated features, integers, steps or components, but do not preclude the presence or addition of one or more other features, integers, steps, components or groups thereof.

It is appreciated that certain features of the invention, which are, for clarity, described in the context of separate embodiments, may also be provided in combination in a single embodiment. Conversely, various features of the invention which are, for brevity, described in the context of a single embodiment, may also be provided separately or in any suitable sub-combination.

The embodiments described above are provided by way of examples only, and various other modifications will be apparent to persons skilled in the field without departing from the scope of the invention herein described and defined.

## Claims

1. An incubator (10) for regulating the temperature of cell culture samples, the incubator (10) comprising:
a support element (12) having a base portion (18) which has an upper lid-facing surface (28) for supporting cell culture samples; and
a lid (14) which covers or substantially covers the support element (12) in an engaged condition;
the base portion (18) of the support element (12) being at least in part thermally conductive at or adjacent to the lid-facing surface (28), and including a layered heater (42) comprising:
a heating element (44); and
a thermal equalisation layer (48) interposed between and being in thermal communication with the heating element (44) and the lid-facing surface (28) of the base portion (18).

2. An incubator (10) as claimed in claim 1, wherein the thermal equalisation layer (48) has anisotropic heat transfer properties within a plane of the lid-facing surface (28) and perpendicular to the said plane.

3. An incubator (10) as claimed in claim 2, wherein the thermal equalisation layer (48) comprises at least in part graphite.

4. An incubator (10) as claimed in any one of claims 1 to 3, wherein the heating element (44) comprises a printed circuit board having heating tracks formed thereon.

5. An incubator (10) as claimed in any one of the preceding claims, further comprising an electrically insulative layer (46) interposed between the heating element (44) and thermal equalisation layer (48).

6. An incubator (10) as claimed in any one of the preceding claims, wherein the support element (12) further comprises at least one cell culture sample positioning element (22).

7. An incubator (10) as claimed in claim 6, wherein the or each cell culture sample positioning element (22) defines a cell culture sample receptacle (24) in which a cell culture sample is receivable.

8. An incubator (10) as claimed in claim 6 or claim 7, further comprising at least one void (32) separating each said cell culture sample positioning element (22) to permit user access to a cell culture sample received therein.

9. An incubator (10) as claimed in any one of claims 6 to 8, further comprising a lower viewing aperture (60) through the base portion (18) of the support element (12) associated with the or each cell culture sample positioning element (22).

10. An incubator (10) as claimed in any one of claims 6 to 9, further comprising an upper viewing aperture (34) through the lid (14), each upper viewing aperture (34) being associated with the or each cell culture sample positioning element (22).

11. An incubator (10) as claimed in any one of the preceding claims, further comprising a thermally conductive layer (68) at or adjacent to a base-facing surface (64) of the lid (14), a further thermal equalisation layer (66) adjacent to the thermally conductive layer (68), and at least one thermal bridge to thermally connect the at least in part thermally conductive base portion (18) of the support element (12) and the thermally conductive layer (68).

12. An incubator (10) as claimed in claim 11 when dependent upon any one of claims 8 to 14, wherein the or each thermal bridge is formed as part of the or each cell culture sample positioning element (22).

13. An incubator (10) as claimed in claim 11 or claim 12, wherein the said further thermal equalisation layer (66) has anisotropic heat transfer properties within a plane of the lid-facing surface (28) and perpendicular to the said plane.

14. A method of maintaining thermal equilibrium within cell culture samples, the method comprising the steps of:
a] providing an incubator (10) as claimed in any one of the preceding claims;
b] activating the heating element (44) to indirectly heat the thermally conductive layer via the thermal equalisation layer (48), the thermal equalisation layer (48) dissipating the thermal energy across its total extent; and
c] heating cell culture samples within the incubator (10) via the thermally conductive layer.

15. A method of optically interrogating cell culture samples within an incubator (10), the method comprising the steps of:
a] providing an incubator (10) having a heating element (44) which includes a thermal equalisation element (48) having at least one viewing aperture therethrough, the thermal equalisation element (48) providing an even temperature through the incubator (10); and
b] providing an optical axis through the incubator (10) associated with the or each viewing aperture; and
c] optically interrogating a cell culture sample on the optical axis inside the incubator (10) using a microscope.
